# FASCICULE DE BREVET EUROPEEN

(11) **EP 1 257 568 B1**
(45) Date de publication et mention de la délivrance du brevet: **24.05.2006**
(21) Numéro de dépôt: 01907783.3
(22) Date de dépôt: 14.02.2001
(51) Int. Cl.: C07K 7/56, A61K 38/12, A61P 31/10

(54) **DERIVES DE L'ECHINOCANDINE, LEUR PROCEDE DE PREPARATION ET LEUR APPLICATION COMME FONGICIDES**
ECHINOCANDIN-DERIVATE, DEREN HERSTELLUNG UND VERWENDUNG ALS FUNGIZID
NOVEL ECHINOCANDIN DERIVATIVES, PREPARATION METHOD AND USE THEREOF AS FUNGICIDES

(30) Priorité: 15.02.2000 FR 0001844
(43) Date de publication de la demande: 20.11.2002
(73) Titulaire: NOVEXEL, 93230 Romainville (FR)
(72) Inventeur: COURTIN, Olivier, F-75012 Paris (FR); DUSSARAT, Arlette, F-93230 Romainville (FR); MELON-MANGUER, Dominique, F-93100 Montreuil (FR); SCHIO, Laurent, F-93140 Bondy (FR)
(74) Mandataire: Hirsch & Associés
(86) Numéro de dépôt international: PCT/FR2001/000419
(87) Numéro de publication internationale: WO 2001/060845

(56) Documents cités:
- WO-A-95/27074
- DATABASE WPI Section Ch, Week 199336 Derwent Publications Ltd., London, GB; Class B02, AN 1993-285439 XP002153622 -& JP 05 202096 A (FUJISAWA PHARM CO LTD (SEE EXAMPLE 21)), 10 août 1993 (1993-08-10)
- ROY E A: "Mulundocandin, a new lipopeptide antibiotic" JOURNAL OF ANTIBIOTICS,JP,JAPAN ANTIBIOTICS RESEARCH ASSOCIATION. TOKYO, vol. 40, no. 3, mars 1987 (1987-03), pages 275-280, XP002129427 ISSN: 0021-8820

## Description

La présente invention concerne de nouveaux dérivés de l'échinocandine, leur procédé de préparation et leur application comme fongicides.

Le brevet JP 5-202096 décrit des dérivés de l'échinocandine liés à une chaîne stéroïde.

L'invention a pour objet les composés répondant à la formule générale (I) : 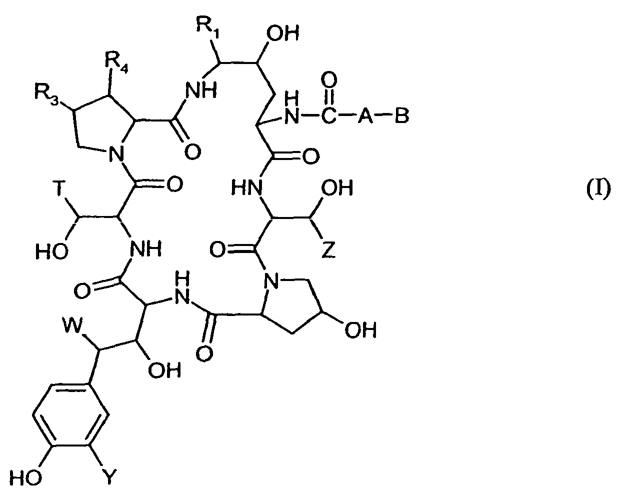 dans lesquels :
R₁ représente un atome d'hydrogène,
un radical hydroxy,
un radical Ob, b représentant un radical alkyle, alkényle ou alkynyle renfermant jusqu'à 12 atomes de carbone, éventuellement substitué par un atome d'halogène, un radical 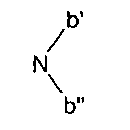
b' et b" identiques ou différents l'un de l'autre représentent un atome d'hydrogène, un radical alkyle renfermant jusqu'à 12 atomes de carbone ou un radical aryle éventuellement substitué,
ou un radical 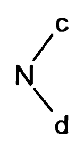 dans lequel :
c et d identiques ou différents l'un de l'autre, représentent un atome d'hydrogène, un radical alkyle renfermant jusqu'à 8 atomes de carbone linéaire, ramifié ou cyclique, éventuellement interrompu par un atome d'oxygène éventuellement substitué par un atome d'halogène, un radical OH, un radical 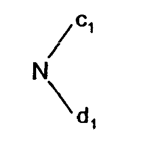
c₁ et d₁ représentant un atome d'hydrogène ou un radical alkyle renfermant jusqu'à 8 atomes de carbone, c₁ et d₁ pouvant éventuellement former avec l'atome d'azote un hétérocycle renfermant éventuellement un ou plusieurs hétéroatomes supplémentaires,
A représente un atome d'oxygène, un radical CH₂ ou un radical NH,
B représente le reste d'un stéroïde,
R₃ représente un atome d'hydrogène, un radical méthyle ou hydroxyle,
R₄ représente un atome d'hydrogène ou un radical hydroxyle,
T représente un atome d'hydrogène, un radical méthyle, un radical CH₂CONH₂, CH₂C N, un radical (CH₂)₂NH₂ ou (CH₂)₂Nalc⁺X⁻, X étant un atome d'halogène et alc un radical alkyle renfermant jusqu'à 8 atomes de carbone,
Y représente un atome d'hydrogène, un radical hydroxyle ou un atome d'halogène ou un radical OSO₃H ou l'un des sels de ce radical,
W représente un atome d'hydrogène ou un radical OH,
Z représente un atome d'hydrogène ou un radical méthyle, et dont les noms suivent :
   1-[(4R,5R)-4,5-dihydroxy-N2-[[[(3β,22E)-ergosta-5,7,22-trièn-3-yl]oxy]carbonyl]-L-ornithine]-déoxymulundocandine (composé 1),
   1-[(4R,5R)-4,5-dihydroxy-N2-[[[(3β)-lanosta-8,24-dièn-3-yl]oxy]carbonyl]-L-ornithine]-déoxymulundocandine (composé 2),
   1-[(4R,SR)-4,S-dihydroxy-N2-[[[(3(3,22E)-ergosta-5,7,22-trièn-3-yl]oxy]carbonyl]-L-omithine]-mulundocandine (composé 3),
   1-[(4R,5R)-4,5-dihydroxy-N2-[[[(3β)-lanosta-8,24-dièn-3-yl]oxy]carbonyl]-L-ornithine]-mulundocandine (composé 4),
   1-[(4R,5R)-4,5-dihydroxy-N2-[[[(3β)-eholest-5-en-3-yl]oxy]carbonyl]-L-ornithine]-mulundocandine (composé 5),
   1-[(4R,5R)-4,5-dihydroxy-N2-[[[(3β)-cholest-5-en-3-yl]oxy]carbonyl]-L-ornithine]-déoxymulundocandine (composé 6),
   1-[(4R,5R)-4,5-dihydroxy-N2-[[[(3β)-cholestan-3-yl]oxy]carbonyl]-L-ornithine]-mulundocandine (composé 7),
   1-[(4R,5R)-4,5-dihydroxy-N2-[[[(3β)-cholestan-3-yl]oxy]carbonyl]-L-ornithine]-déoxymulundocandine (composé 8),
   1-[(4R,5R)-4,5-dihydroxy-N2-[[[(3β)-stigmast-5-en-3-yl]oxy]carbonyl]-L-ornithine]-déoxymulundocandine (composé 9),
   1-[(4R,5R)-4,5-dihydroxy-N2-[[[(3β,22e)-stigmasta-5,22-dien-3-yl]oxy]carbonyl]-L-ornithine]-déoxymulundocandine (composé 10),
   1-[(4R,5R)-4,5-dihydroxy-N2-[[[(3β)-stigmast-5-en-3-yl]oxy]carbonyl]-L-ornithine]-mulundocandine (composé 11),
   1-[(4R,5R)-4,5-dihydroxy-N2-(cholestan-3-ylacétyl)-L-ornithine]-déoxymuiundocandine (isomère A) et (isomère B) (composé 12), et
   1-[(4R,5R)-4,5-dihydroxy-N2-[[[(3β,22E)-stigmasta-5,22-dièn-3-yl]oxy]carbonyl]-L-ornithine]-mulundocandine, (composé 13),
   ainsi que les sels d'addition avec les acides de ces produits de formule (I).

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

L'invention a tout particulièrement pour objet les composés 1, 2 et 3.

Les composés de la présente invention présentent d'intéressantes propriétés antifongiques ; ils sont notamment actifs sur *Candida albicans* et autres *Candida* comme *Candida glabrata, krusei, tropicalis, pseudotropicalis, parapsilosis et Aspergillus fumigatus, Aspergillus flavus, Cryptococcus neoformans*.

Les composés de la présente invention peuvent être utilisés en tant que médicaments chez l'homme ou l'animal, pour lutter notamment contre les candidoses digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.

Les composés de l'invention peuvent être utilisés également dans la prévention des affections mycosiques chez les déprimés immunitaires congénitaux ou acquis.

Les composés de l'invention ne sont pas limités à une utilisation pharmaceutique, ils peuvent être également utilisés comme fongicides dans d'autres domaines que pharmaceutiques.

L'invention a donc pour objet à titre de composés antifongiques, les composés de l'invention.

L'invention a également pour objet les composés de l'invention, à titre de médicaments.

L'invention a tout particulièrement pour objet les compositions pharmaceutiques renfermant comme principe actif au moins un composé de l'invention ou l'un de ses sels.

Ces compositions peuvent être administrées par voie buccale, rectale, parentérale ou par voie locale en application topique sur la peau et les muqueuses, mais la voie préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour les produits des exemples 1, 2 et 3.

L'invention a également pour objet un procédé de préparation des composés de l'invention, caractérisé en ce que l'on soumet un composé de formule (II) : 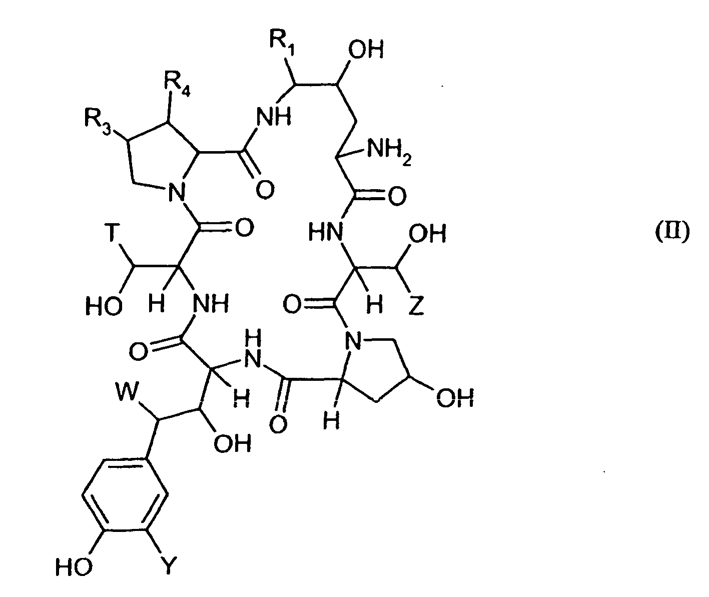 à l'action d'un stéroïde BOH ou d'un de ses dérivés, le composé de formule (II) et le stéroïde BOH étant choisis de manière appropriée, en fonction du composé selon l'invention que l'on souhaite préparer, en présence d'un activateur de la fonction alcool ou d'un acide correspondant ou d'un de ses dérivés, pour obtenir le composé de l'invention correspondant.

Les composés de formule (II) utilisés comme produit de départ sont des produits connus décrits par exemple dans la demande de brevet européen 2772028.

Dans un mode de réalisation préféré du procédé de l'invention on fait réagir le composé (II) avec un alcool dont la fonction alcool est activée, par exemple à l'aide du diphosgène ou un acide dont la fonction acide est activée par exemple à l'aide d'un chloroformiate d'alkyle, comme il sera détaillé ci-après dans la partie expérimentale.

### COMPOSE 1: 1-[(4R,5R)-4,5-dihydroxy-N2-[[[(3β,22E)-ergosta-5,7,22-trièn-3-yl]oxy]carbonyl]-L-ornithine]-déoxymulundocandine,

### Stade A :

On ajoute 176 µl de triéthylamine dans une solution renfermant 500 mg d'ergostérol et 10 ml de chlorure de méthylène. On ajoute ensuite à -20°C, 152 µl de diphosgène en solution dans 200 µl de chlorure de méthylène. On maintient le mélange réactionnel sous agitation pendant 3 heures à une température comprise entre 0 et 10°C. On amène à sec le produit obtenu.

### Stade B :

On introduit à -5°C le produit préparé au stade A dans un mélange renfermant 627,3 mg de nucleus de déoxymulundocandine, 30 ml de dioxanne, 109,4 mg de carbonate acide de sodium et 2,3 ml d'eau déminéralisée. On maintient le mélange réactionnel sous agitation pendant 18 heures, puis concentre sous pression réduite. On chromatographie le produit obtenu sur silice en éluant avec le mélange chlorure de méthylène/ méthanol/eau (86-13-1). On obtient 73 mg de produit que l'on reprend dans l'éther en présence de quelques gouttes d'acétate d'éthyle, essore, lave à l'éther et sèche. On obtient 69 mg de produit que l'on reprend dans un minimum d'eau, triture, essore et lave à l'eau et à l'éther. On sèche et obtient 31,2 mg de produit.
CCM (CH₂Cl₂/CH₃OH/H₂O : 86-13-1) Rf = 0,12.
Spectre de masse : MNa⁺ = 1212⁺
MH⁺ = 293⁺

| Résidu | δppm1H | δppm13C |
|---|---|---|
| A | Thréonine | (Thr) |
| NH | 8,08 | - |
| C=O | - | 169,4 |
| CHα | 4,78 | 56,2 |
| HO-CHβ | 4,51 | 64,7 |
| CH₃γ | 1,02 | 19,3 |

| | | |
|---|---|---|
| B | γHydroxy proline | (OH Pro) |
| δN | - | - |
| C=O | - | 170,9 |
| CHα | 4,31 | 60,9 |
| CH₂β | 1,92-2,19 | 37,1 |
| HO-CHγ | 4,40 | 68,9 |
| NCH₂ | 4,16-3,60 | 55,4 |
| C | βHydroxy homo tyrosine | (hTyr) |
| NH | 7,59 | - |
| C=O | - | 169,2 |
| CHα | 4,21 | 55,6 |
| HO-CHβ | 4,09 | 72,5 |
| CH₂γ | 2,48-2,57 | 38,7 |
| Cl | - | 128,1 |
| C2-6 | 6,94 | 129,8 |
| C3-5 | 6,64 | 114,4 |
| C4 | - | 155,1 |
| OH | 9,12 | - |

| D | Sérine | (Ser) |
|---|---|---|
| NH | 7,30 | - |
| C=O | - | 167,8 |
| CHα | 4,85 | 51,5 |
| HO-CH₂β | 3,45-3,57 | 62,0 |

| | | |
|---|---|---|
| E | βHydroxy γmétyl proline | (OH Me Pro) |
| N | - | - |
| C=O | - | 168,4 |
| CHα | 4,16 | 68,6 |
| HO-CHβ | 3,99 | 73,6 |
| CHγ | 2,34 | 36,7 |
| CH₃ | 0,98 | 10,4 |
| NCH₂ | 3,23-3,91 | 50,8 |

| | | |
|---|---|---|
| F/F' | Omithine | (Orn) |
| F NH | 7,79 | - |
| HO-CHα | 5,10 | 71,4 |
| HO-CHβ | 3,83 | 69,2 |
| F' NH | 7,17 | - |
| C=O | - | 171,5 |
| CHα | 3,86 | 51,3 |
| CH₂β | 1,68-1,82 | 33,9 |

| | | |
|---|---|---|
| S | ¹H | ¹³C |
| 3 | 4,34 | 71,7 |
| 4 | 2,26-2,50 | 36,4 |
| 6 | 5,54 | 119,3 |
| 7 | 5,34 | 115,8 |
| 9 | 1,93 | 45,2 |
| 14 | 1,87 | 53,6 |
| 17 | 1,26 | 54,8 |
| 18Me | 0,58 | 11,6 |

| | | |
|---|---|---|
| 19Me | 0,90 | 15,5 ou 17,0 |
| 20 | 2,02 | 39,5 |
| 21 | 1,01 | 20,5 |
| 22 | 5,21 | 135,0 |
| 23 | 5,22 | 131,2 |
| 24 | 1,83 | 41,7 |
| 25 | 1,46 | 32,1 |
| 26 | 0,81 | 19,3 |
| 27 | 0,81 | 19,3 |
| 28 | 0,89 | 15,5 ou 17,0 |

| | | |
|---|---|---|
| S | ¹H | ¹³C |
| Les autres CH₂ | 1,65-1,58 | 20,1 |
| | 1,61-1,29 | 22,1 |
| | 1,71-1,27 | 27,5 |
| | 1,81-1,50 | 27,9 |
| | 1,83-1,28 | 37,2 |
| | 2,01-1,23 | 38,2 |

### COMPOSE 2 : 1-[(4R,5R)-4,5-dihydroxy-N2-[[[(3β)-lanosta-8,24-dièn-3-yl]oxy]carbonyl]-L-ornithine]-déoxymulundocandine,

### Stade A :

On introduit 73 µl de diphosgène et 84 µl de triéthylamine dans une solution renfermant 256 mg de lanostérol et 4,6 ml de chlorure de méthylène.

### Stade B :

On introduit dans un mélange de 238 mg de déoxymulundocandine et 19 ml de dioxane, 50,49 mg de carbonate acide de sodium et 1,1 ml d'eau. On refroidit à -5°C, introduit le produit préparé au stade A. On agite 10 minutes à -5°C et laisse la température remonter à la température ambiante. On agite pendant 15 heures à la température ambiante. On chromatographie le produit obtenu CH₂Cl₂/CH₃OH/H₂O (86-13-1). On obtient 75 mg de produit que l'on chromatographie sur silice en éluant avec le mélange chlorure de méthylène/méthanol/eau (84-15-1). On obtient 41,7 mg de produit. Spectre de masse : MNa⁺ = 1242,5

### COMPOSE 3: 1-[(4R,SR)-4,5-dihydroxy-N2-[[[(3β,22E)-ergosta-5,7,22-trièn-3-yl]oxy]carbonyl]-L-ornithine]-mulundocandine,

### Stade A :

On ajoute 95 µl de triéthylamine, dans une solution renfermant 224 mg d'ergostérol et 7 ml de chlorure de méthylène. On refroidit la solution obtenue à -5°C et ajoute 75 µl de trichlorométhylchloroformiate. On agite la suspension à [0°C + 5°C] pendant 3 heures. On amène à sec sous pression réduite et laisse une heure sous vide. On obtient un produit que l'on utilise tel quel au stade B.

### Stade B :

On agite pendant 30 minutes, une suspension renfermant 250 mg de nucleus de mulundocandine et 20 ml de dioxanne. On ajoute 59 mg de carbonate acide de sodium en solution dans 1,5 ml d'eau. On refroidit la suspension à 0°C + 5°C et ajoute le produit obtenu au stade A. On laisse revenir à la température ambiante et agite pendant 20 heures. On amène à sec, sous pression réduite. On reprend avec 5 ml d'acétate d'éthyle, triture et essore. On obtient 125 mg de produit que l'on chromatographie sur silice en éluant avec le mélange chlorure de méthylène/méthanol/eau (79-20-1). On obtient ainsi le produit recherché.
Spectre de masse SM : 1228 MNa⁺

| Résidu | δppm1H | δppm13C |
|---|---|---|
| A | Thréonine | (Thr) |
| NH | 7,96 | - |
| C=O | - | 169,9 |
| CHα | 4,78 | 56,0 |
| HO-CHβ | 4,40 | 65,5 |
| CH₃γ | 1,08 | 19,2 |

| B | γHydroxy proline | (OH Pro) |
|---|---|---|
| N | - | - |
| C=O | - | 170,2 |
| CHα | 4,31 | 60,5 |
| CH₂β | 1,86-2,18 | 36,9 |
| HO-CHγ | 4,40 | 68,9 |
| NCH₂δ | 3,66-3,90 | 55,3 |

| C | βHydroxy homo tyrosine | (hTyr) |
|---|---|---|
| NH | 7,33 | - |
| C=O | - | 169,3 |
| CHα | 4,03 | 53,4 |
| HO-CHβ | 3,98 | 74,9 |
| HO-CHβ | 4,21 | 72,9 |
| C1 | - | 132,2 |
| C2-6 | 7,02 | 127,7 |
| C3-5 | 6,66 | 114,4 |
| C4 | - | 156,3 |
| OH | 9,23 | - |

| D | Sérine | (Ser) |
|---|---|---|
| NH | 7,29 | - |
| C=O | - | 167,9 |
| CHα | 4,80 | 51,8 |
| HO-CH₂β | 3,52 | 62,1 |
| E | βHydroxy γméthyl proline | (OH Me Pro) |
| N | - | - |
| C=O | - | 168,4 |
| CHα | 4,18 | 68,5 |
| HO-CHβ | 3,99 | 73,4 |
| CHγ | 2,34 | 36,6 |
| CH₃δ | 0,96 | 10,5 |
| NCH₂δ | 3,20-3,90 | 50,8 |

| F/F' | Ornithine | (Orn) |
|---|---|---|
| F NH | 7,82 | - |
| HO-CHα | 5,08 | 71,7 |
| HO-CHβ | 3,80 | 69,0 |
| F' NH | 7,18 | - |
| C=O | - | - |
| CHα | 3,89 | 51,5 |
| CH₂β | 1,82-1,68 | 34,1 |

| R | ¹H | ¹³C |
|---|---|---|
| C=O | - | - |
| 1 | - | - |
| 2 | - | - |
| 3 | 4,35 | 71,7 |
| 4 | 2,27-2,50 | 36,4 |
| 5 | - | 138,5 |
| 6 | 5,54 | 119,4 |
| 7 | 5,34 | 115,8 |
| 8 | - | 146,3 |
| 9 | 1,95 | 45,2 |
| 10 | - | - |
| 11 | 1,26 | 21,7 |
| 12 | 2,02 | 38,1 |
| 13 | - | 42,1 |
| 14 | 1,89 | 53,5 |
| 15 | - | - |
| 16 | - | - |
| 17 | 1,29 | 54,7 |
| 18Me | 0,60 | 11,4 |
| 19Me | 0,89 | 15,4 |
| 20 | 2,04 | 39,5 |
| 21Me | 1,01 | 20,7 |
| 22 | 5,21 | 135,0 |
| 23 | 5,23 | 131,0 |
| 24 | 1,87 | 41,7 |
| 25 | 1,48 | 32,1 |
| 26 | 0,80-0,82 | 19,2 |
| 27 | - | - |
| 28 | 0,89 | 16,9 |

### COMPOSE 4 : 1-[(4R,5R)-4,5-dihydroxy-N2-[[[(3β)-lanosta-8,24-dièn-3-yl]oxy]carbonyl]-L-ornithine]-mulundocandine,

En opérant comme précédemment à partir de 256 mg de lanostérol et 235 mg de nucleus de déoxymulundocandine, on a obtenu 45 mg de produit recherché.
Spectre de masse : 12580 MNa⁺

### COMPOSE 5: 1-[(4R,5R)-4,5-dihydroxy-N2-[[[(3β)-cholest-5-en-3-yl]oxy]carbonyl]-L-ornithine]-mulundocandine,

En opérant comme précédemment, à partir de 233 mg de cholestérol et de 250 mg de nucleus de mulundocandine, on a obtenu 10 mg de produit recherché.
SM : MH⁺ = 1197,3
MNa⁺ = 1218,0

### COMPOSE 6 : 1-[(4R,5R)-4,5-dihydroxy-N2-[[[(3β)-cholest-5-en-3-yl]oxy]carbonyl]-L-ornithine]-déoxymulundocandine,

En opérant comme au composé 1, à partir de 233 mg de cholestérol et de 250 mg de déoxymulundocandine, on a obtenu le produit recherché.
SM : MNa⁺ = 12202

### COMPOSE 7 : 1-[(4R,5R)-4,5-dihydroxy-N2-[[[(3β)-cholestan-3-yl]oxy]carbonyl]-L-ornithine]-mulundocandine,

En opérant comme au composé 1, à partir de 455 mg de 5 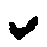 -cholestane-3 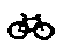 -ol, on a obtenu 521 mg de produit recherché.

### COMPOSE 8 : 1-[(4R,5R)-4,5-dihydroxy-N2-[[[(3β)-cholestan-3-yl]oxy]carbonyl]-L-ornithine]-déoxymulundocandine,

En opérant comme au composé 1 à partir de 455 mg de 5 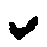 -cholestan 3 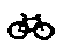 -ol et de 449,93 mg de déoxymulundocandine, on a obtenu 186 mg de produit recherché.
SM : 1204,4 Da = MNa⁺

### COMPOSE 9 : 1-[(4R,5R)-4,5-dihydroxy-N2-[[[(3β)-stigmast-5-en-3-yl]oxy]carbonyl]-L-ornithine]-déoxymulundocandine,

En opérant comme au composé 1 à partir de 829 mg de 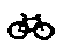 -sitostérol et 1,2 g de nucleus de déoxymulundocandine, on a obtenu 99 mg de produit recherché.
Spectre de masse : 1230 Da = MNa⁺

### COMPOSE 10 : 1-[(4R,5R)-4,5-dihydroxy-N2-[[[(3β,22e)-stigmasta-5,22-dien-3-yl]oxy]carbonyl]-L-ornithine]-déoxymulundocandine,

En opérant comme au composé 1 à partir de 300 mg de stigmastérol et 395 mg de nucleus de déoxymulundocandine, on obtient 53 mg de produit recherché.
Spectre de masse : 1228 Da = MNa⁺

### COMPOSE 11 : 1-[(4R,5R)-4,5-dihydroxy-N2-[[[(3β)-stigmast-5-en-3-yl]oxy]carbonyl]-L-ornithine]-mulundocandine,

En opérant comme au composé 1, à partir de 810 mg de 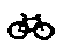 -sitostérol et de 500 mg de nucleus de mulundocandine, on a obtenu 330 mg de produit recherché.
Spectre de masse : 1230 Da = MLi⁺

### COMPOSE 12 : 1-[(4R,5R)-4,5-dihydroxy-N2-(cholestan-3-ylacétyl)-L-ornithine]-déoxymulundocandine (isomère A) et (isomère B)

On ajoute 44 µl de tributylamine dans une solution renfermant 67,8 mg du produit de la préparation P ci-dessous et un mélange de 1,5 ml de DMF et 1,5 ml de dioxanne. On porte au bain de glace et ajoute 22 ml de chloroformiate d'isobutyle. On maintient le mélange réactionnel 10 minutes au bain de glace, puis 2 heures à la température ambiante. On ajoute 100 mg de nucleus de déoxymulundocandine. On maintient sous agitation pendant 15 heures. On concentre sous pression réduite, reprend à l'éther, triture et essore. On lave à l'éther et obtient 177 mg de produit que l'on chromatographie sur silice en éluant avec le mélange chlorure de méthylène/méthanol/eau (86-13-11). On obtient 19 mg d'isomère A et 47 mg d'isomère B.
Rf = 0,47 et Rf = 0,43.

### PREPARATION P :

### Stade A :

On ajoute 2,47 ml de n-butyllithium dans l'hexane à 15 % dans une solution renfermant 961 mg de diméthoxyphosphonoacétate de méthyle et 2 ml de THF. On agite pendant 30 minutes et ajoute 500 mg de cholestanone et 2 ml de THF anhydre. On agite pendant 1 heure, ajoute 2 ml d'eau et extrait à l'éther. On lave à l'eau, sèche sur sulfate de magnésium, filtre et amène à sec. On reprend le résidu dans le méthanol, triture et essore. On obtient 544 mg de produit recherché.

### Stade B :

On maintient sous agitation et atmosphère d'hydrogène pendant 15 heures, 523 mg du produit du stade précédent, 105 mg de palladium sur charbon à 10 % et 20 ml de cyclohexane. On filtre et amène à sec. On obtient 520 mg de produit que l'on chromatographie sur silice en éluant avec le mélange cyclohexane/acétate d'éthyle (95-5) puis avec le mélange cyclohexane/chlorure de méthylène (5/5). On obtient un mélange de 2 isomères. Rf = 0,49 et Rf = 0,67.

### Stade C :

On ajoute 200 µl de lessive de soude à une suspension renfermant 502 mg de produit du stade précédent, 2 ml d'éthylène glycol. On porte le mélange réactionnel au reflux pendant 50 minutes. On refroidit à 50°C, acidifie avec de l'acide chlorhydrique, concentre. On ajoute 2,6 ml d'eau, porte la solution au reflux, essore et lave à l'eau, dissout le produit obtenu dans le chlorure de méthylène. On décante, sèche et amène à sec. On obtient 460 mg de produit recherché. Rf = 0,34 CH₂Cl₂/MEOH (95-5).

### COMPOSE 13 : 1-[(4R,SR)-4,5-dihydroxy-N2-[[[(3β,22E)-stigmasta-5,22-dièn-3-yl]oxy]carbonyl]-L-ornithine]-mulundocandine,

En opérant comme au composé, à partir de 403 mg de stigmastérol et de 400 mg de nucleus de mulundocandine, on obtient 176 mg de produit recherché.

### EXEMPLE : Composition pharmaceutique

On a préparé des comprimés renfermant :
- composé 1 150 mg
- Excipient q.s.p. 1 g
(Détail de l'excipient : amidon, talc, stéarate de magnésium).

### ETUDE PHARMACOLOGIQUE

### A - Inhibition de la glucane synthase de Candida albicans.

On purifie des membranes de Candida albicans selon le procédé décrit par Tang et al Antimicrob. Agents Chemother 35, 99-103, 1991. 22,5 µg de protéines membranaires sont incubées dans un mélange de 2 mM de ¹⁴C-UDP glucose (activité spécifique = 0,34 mCi./mmol, 50 µg d'α-amylase, 1 mM de dithiotreitol (DTT), 1 mM EDTA, 100 mM NaF, 7µM de GTP-γ-S, 1 mM de sucrose et 50 mM de Tris-HCL (pH 7,8) dans un volume de 100 µl. Le milieu est incubé à 25°C pendant 1 heure et la réaction terminée par addition de TCA à une concentration finale de 5 %. Le mélange réactionnel est transféré sur un filtre de fibre de verre pré-humidifié. Le filtre est lavé, séché et sa radioactivité est comptée.

La mulundocandine est utilisé comme contrôle positif

Le contrôle du véhicule est effectué avec la même quantité de DMSO 1 %. Les résultats obtenus montrent que les produits de l'invention présentent sur ce test une bonne activité en particulier les composés 2 et 3.

### B - activité sur l'enzyme d'Aspergillus fumigatus.

L'enzyme est préparée selon le procédé de Beaulieu et al.(Antimicrob. Agents Chemother 38, 937-944, 1994).

Le protocole utilisé est identique au protocole décrit ci-dessus pour l'enzyme de Candida albicans sauf que l'on n'utilise pas de dithiotreitol dans le mélange réactionnel.

Les produits de l'invention présentent une bonne activité sur ce test.

## Revendications

1. Les dérivés de l'échinocandine dont les noms suivent :
1-[(4R,5R)-4,5-dihydroxy-N2-[[[(3β,22E)-ergosta-5,7,22-trièn-3-yl]oxy]carbonylJ-L-ornithine]-déoxymulundocandine,
1-[(4R,5R)-4,5-dihydroxy-N2-[[[(3β)-lanosta-8,24-dièn-3-yl]oxy]carbonyl]-L-ornithine]-déoxymulundocandine
1-[(4R,5R)-4,5-dihydroxy-N2-[[[(3β,22E)-ergosta-5,7,22-trièn-3-yl]oxy]carbonyl]-L-ornithine]-mulundocandine
1-[(4R,5R)-4,5-dihydroxy-N2-[[[(3β)-lanosta-8,24-dièn-3-yl]oxy]carbonyl]-L-ornithine]-mulundocandine
1-[(4R,5R)-4,5-dihydroxy-N2-[[[(3β)-cholest-5-en-3-yl]oxy]carbonyl]-L-ornithine]-mulundocandine
1 -[(4R,5R)-4,5-dihydroxy-N2-[[[(3β)-cholest-5-en-3-yl]oxy]carbonyl]-L-ornithine]-déoxymulundocandine
1-[(4R,5R)-4,5-dihydroxy-N2-[[[(3β)-cholestan-3-yl]oxy]carbonyl]-L-ornithine]-mulundocandine
1-[(4R,5R)-4,5-dihydroxy-N2-[[[(3β)-cholestan-3-yl]oxy]carbonyl]-L-ornithine]-déoxymulundocandine
1-[(4R,5R)-4,5-dihydroxy-N2-[[[(3β)-stigmast-5-en-3-yl]oxy]carbonyl]-L-ornithine]-déoxymulundocandine
1-[(4R,5R)-4,5-dihydroxy-N2-[[[(3β,22e)-stigmasta-5,22-dien-3-yl]oxy]carbonyl]-L-ornithine]-déoxymulundocandine
1-[(4R,5R)-4,5-dihydroxy-N2-[[[(3β)-stigmast-5-en-3-yl]oxy]carbonyl]-L-ornithine]-mulundocandine
1-[(4R,5R)-4,5-dihydroxy-N2-(cholestan-3-ylacétyl)-L-omithine]-déoxymulundocandine (isomère A) et (isomère B)
1-[(4R,5R)-4,5-dihydroxy-N2-[[[(3β,22E)-stigmasta-5,22-dièn-3-yl]oxy]carbonyl]-L-ornithine]-mulundocandine,
ainsi que les sels d'addition avec les acides de ces dérivés.

2. Les composés selon la revendication 1 dont les noms suivent :
1-[(4R,5R)-4,5-dihydroxy-N2-[[[(3β,22E)-ergosta-5,7,22-trièn-3-yl]oxy]carbonyl]-L-ornithine]-mulundocandine,
1-[(4R,5R)-4,5-dihydroxy-N2-[[[(3β)-lanosta-8,24-dièn-3-yl] oxy]carbonyl]-L-ornithine]-déoxymulundocandine,
1-[(4R,5R)-4,5-dihydroxy-N2-[[[(3β,22E)-ergosta-5,7,22-trièn-3-yl]oxy]carbonyl]-L-ornithine]-déoxymulundocandine.

3. A titre de composés antifongiques, les composés selon la revendication 1 ou 2.

## Claims

1. The echinocandin derivatives the names of which follow:
- 1-[(4R,5R)-4,5-dihydroxy-N2-[[[(3β,22E)-ergosta-5,7,22-trien-3-yl]oxy]carbonyl]-L-ornithine]-deoxymulundocandin,
- 1-[(4R,5R)-4,5-dihydroxy-N2-[[[(3β)-lanosta-8,24-dien-3-yl]oxy]carbonyl]-L-ornithine]-deoxymulundocandin,
- 1-[(4R,5R)-4,5-dihydroxy-N2-[[[(3β,22E)-ergosta-5,7,22-trien-3-yl]oxy]carbonyl]-L-omithine]-mulundocandin,
- 1-[(4R,5R)-4,5-dihydroxy-N2-[[[(3β)-lanosta-8,24-dien-3-yl]oxy]carbonyl]-L-ornithine]-mulundocandin,
- 1-[(4R,5R)-4,5-dihydroxy-N2-[[[(3β)-cholest-5-en-3-yl]oxy]carbonyl]-L-ornithine]-mulundocandin,
- 1-[(4R,5R)-4,5-dihydroxy-N2-[[[(3β)-cholest-5-en-3-yl]oxy]carbonyl]-L-ornithine]-deoxymulundocandin,
- 1-[(4R,5R)-4,5-dihydroxy-N2-[[[(3β)-cholestan-3-yl]oxy]carbonyl]-L-ornithine]-mulundocandin,
- 1-[(4R,5R)-4,5-dihydroxy-N2-[[[(3β)-cholestan-3-yl]oxy]carbonyl]-L-ornithine]-deoxymulundocandin,
- 1-[(4R,5R)-4,5-dihydroxy-N2-[[[(3β)-stigmast-5-en-3-yl]oxy]carbonyl]-L-ornithine]-deoxymulundocandin,
- 1-[(4R,5R)-4,5-dihydroxy-N2-[[[(3β,22e)-stigmasta-5,22-dien-3-yl]oxy]carbonyl]-L-ornithine]-deoxymulundocandin,
- 1-[(4R,5R)-4,5-dihydroxy-N2-[[[(3β)-stigmast-5-en-3-yl]oxy]carbonyl]-L-ornithine]-mulundocandin,
- 1-[(4R,5R)-4,5-dihydroxy-N2-(cholestan-3-ylacetyl)-L-ornithine]-deoxymulundocandin (isomer A) and (isomer B)
- 1-[(4R,5R)-4,5-dihydroxy-N2-[[[(3β,22E)-stigmasta-5,22-dien-3-yl]oxy]carbonyl]-L-ornithine]-mulundocandin,
as well as the addition salts with acids of these derivatives.

2. The compounds according to claim 1 the names of which follow:
1-[(4R,5R)-4,5-dihydroxy-N2-[[[(3β,22E)-ergosta-5,7,22-trien-3-yl]oxy]carbonyl]-L-ornithine]-mulundocandin,
1-[(4R,5R)-4,5-dihydroxy-N2-[[[(3β)-lanosta-8,24-dien-3-yl] oxy]carbonyl]-L-ornithine]-deoxymulundocandin,
1-[(4R,5R)-4,5-dihydroxy-N2-[[[(3β,22E)-ergosta-5,7,22-trien-3-yl]oxy]carbonyl]-L-ornithine]-deoxymulundocandin.

3. As antifungal compounds, the compounds according to claim 1 or 2.

## Patentansprüche

1. Echinocandin-Derivate der folgenden Bezeichnungen:
1-[(4R,5R)-4,5-Dihydroxy-N2-[[[(3β,22E)-ergosta-5,7,22-trien-3-yl]-oxy]-carbonyl]-L-ornithin]-desoxymulundocandin,
1-[(4R,5R)-4,5-Dihydroxy-N2-[[[(3β)-lanosta-8,24-dien-3-yl]-oxy]-carbonyl]-L-ornithin]-desoxymulundocandin,
1-[(4R,5R)-4,5-Dihydroxy-N2-[[[(3β,22E)-ergosta-5,7,22-trien-3-yl]-oxy]-carbonyl]-L-ornithin]-mulundocandin,
1-[(4R,5R)-4,5-Dihydroxy-N2-[[[(3β)-lanosta-8,24-dien-3-yl]-oxy]-carbonyl]-L-ornithin]-mulundocandin,
1-[(4R,5R)-4,5-Dihydroxy-N2-[[[(3β)-cholest-5-en-3-yl]-oxy]-carbonyl]-L-ornithin]-mulundocandin,
1-[(4R, 5R)-4, 5-Dihydroxy-N2-[[[(3β)-cholest-5-en-3-yl]-oxy]-carbonyl]-L-ornithin]-desoxymulundocandin,
1-[(4R,5R)-4,5-Dihydroxy-N2-[[[(3β)-cholestan-3-yl]-oxy]-carbonyl]-L-ornithin]-mulundocandin,
1-[(4R,5R)-4,5-Dihydroxy-N2-[[[(3β)-cholestan-3-yl]-oxy]-carbonyl]-L-ornithin]-desoxymulundocandin,
1-[[4R,5R)-4,5-Dihydroxy-N2-[[[(3β)-stigmast-5-en-3-yl]-oxy]-carbonyl]-L-ornithin]-desoxymulundocandin,
1-[(4R,5R)-4,5-Dihydroxy-N2-[[[(3β,22e)-stigmasta-5,22-dien-3-yl]-oxy]-carbonyl]-L-ornithin]-desoxymulundocandin.
1-[(4R,5R)-4,5-Dihydroxy-N2-[[[(3β)-stigmast-5-en-3-yl]-oxy]-carbonyl]-L-ornithin]-mulundocandin,
1-[(4R,5R)-4,5-Dihydroxy-N2-(cholestan-3-ylacetyl)-L-ornithin]-desoxymulundocandin (Isomeres A) und (Isomeres B),
1-[(4R,5R)-4,5-Dihydroxy-N2-[[[(3β,22E)-stigmasta-5,22-dien-3-yl]-oxy]-carbonyl]-L-ornithin]-mulundocandin
sowie die Säureadditionssalze dieser Derivate.

2. Verbindungen nach Anspruch 1 der folgenden Bezeichnungen:
1-[(4R,5R)-4,5-Dihydroxy-N2-[[[(3β,22E)-ergosta-5,7,22-trien-3-yl]-oxy]-carbonyl]-L-ornithin]-mulundocandin,
1-[(4R,5R)-4,5-Dihydroxy-N2-[[[(3β)-lanosta-8,24-dien-3-yl]-oxy]-carbonyl]-L-ornithin]-desoxymulundocandin,
1-[(4R,5R)-4,5-Dihydroxy-N2-[[[(3β,22E)-ergosta-5,7,22-trien-3-yl]-oxy]-carbonyl]-L-ornithin]-desoxymulundocandin.

3. Verbindungen nach den Ansprüchen 1 oder 2 als antifungistische Verbindungen.
